# EUROPEAN PATENT SPECIFICATION

(11) **EP 1 441 667 B1**
(45) Date of publication and mention of the grant of the patent: **17.01.2007**
(21) Application number: 02777331.6
(22) Date of filing: 29.10.2002
(51) Int. Cl.: A61F 2/04, A61K 31/505, A61M 25/04

(54) **IMPROVED ENDOPROSTHETIC DEVICE**
VERBESSERTE ENDOPROTHETISCHE VORRICHTUNG
DISPOSITIF AMELIORE D'ENDOPROTHESE

(30) Priority: 30.10.2001 EP 01125840
(43) Date of publication of application: 04.08.2004
(73) Proprietor: Boehringer Ingelheim Pharma GmbH & Co.KG, 55216 Ingelheim am Rhein (DE)
(72) Inventor: EISERT, Wolfgang, 30175 Hannover (DE)
(86) International application number: PCT/EP2002/012054
(87) International publication number: WO 2003/037220

(56) References cited:
- WO-A-98/20928
- J.L.YU ET AL.: "Promotion of Escherichia coli adherence to rubber slices by adsorbed fibronectin" THE JOURNAL OF MEDICAL MICROBIOLOGY, vol. 41, no. 2, August 1994 (1994-08), pages 133-138, XP009006915

## Description

### FIELD OF THE INVENTION

The present invention relates to an improved endoprosthetic device for insertion in a vessel and for administration of a therapeutic compound.

### BACKGROUND OF THE INVENTION

Endoprosthetic devices known as stents are placed or implanted within a vessel for treating problems such as stenoses, strictures, or aneurysms in the vessel. Typically, these devices are implanted in a vessel to reinforce collapsing, partially occluded, weakened or dilated vessels. Stents may also be implanted in the urethra, ureter, bile duct, or any body vessel which has been narrowed or weakened.

Stents made of various materials including metals, alloys and plastics and formed into variety of geometric shapes have been described in the art. Two types of stents have been commonly employed. Spring-like or self-expanding stents, formed typically of metals or alloys, are inserted into the target vessel with a restraining element or sheath over the stent, to prevent the stent from expanding until placement at the target site. The other type of stent requires a stimulus to expand the stent after placement at the target vessel. Most often, this stimulus is radial force or pressure applied by inflation of a balloon on a catheter. Stents which respond to other stimuli, such as heat, are also known, and these stents are generally composed of a shape-memory material, either an alloy or a polymer.

It is often desirable to administer a drug at the target site, where the stent also serves as a framework for carrying the therapeutic compound. Numerous approaches have been proposed and, for metal stents, one proposed approach is to directly coat the stent wires with a polymer containing the therapeutic agent. This approach suffers from several problems including cracking of the polymer as the stent is expanded during deployment. Because the stent wires have a limited surface area, and because the overall polymer coating should be thin so that it will not significantly increase the profile of the stent, the amount of polymer that can be applied is limited. Hence, another disadvantage with polymer-coated stents for drug delivery is a limited capacity for carrying a drug.

Another approach to providing delivery of a drug in combination with a stent has been to include a sheath, which encompasses the stent and contains the therapeutic agent. (US 5,383,928; US 5,453,090). Such sheaths are typically secured to the stent by means of a hemostat or other clamping mechanism, which have the disadvantage of increasing the profile of the catheter, reducing flexibility and tractability.

However major problem is that stents induce a vascular smooth muscle cell proliferation, leading to significant restenosis within few months.

### SUMMARY OF THE INVENTION

Accordingly, it is an object of the invention to provide a stent which overcomes the above-mentioned problems. It has now been found surprisingly that the vascular smooth cell proliferation caused by stents can be reduced, if said stent comprises a pyrimidino-pyrimidine compound.

Dipyridamole {2,6-bis(diethanolamino)-4,8-dipiperidino-pyrimido[5,4-d]pyrimidine}, closely related substituted pyrimido-pyrimidines and their preparation have been described in e.g. U.S.Patent 3,031,450. Further related substituted pyrimido-pyrimidines and their preparation have been described in e.g. GB 1,051,218, inter alia the compound mopidamol {2,6-bis-(diethanolamino)-4-piperidinopyrimido[5,4-d]pyrimidine}.

Dipyridamole was introduced as a *coronary vasodilator* in the early 1960s. It is also well known having *platelet aggregation inhibitor properties* due to the inhibition of adenosine uptake. Subsequently, dipyridamole was shown to reduce thrombus formation in a study of arterial circulation of the brain in a rabbit model. These investigations led to its use as an *antithrombotic agent;* it soon became the therapy of choice for such applications as stroke prevention, maintaining the patency of coronary bypass and valve-replacement, as well as for treatment prior to coronary angioplasty.

The European patent application EP 0 543 653 suggests the use of dipyridamole for the preparation of a formulation adapted for local delivery to proliferative cells. There is no hint to stents comprising dipyridamole at all.

Mopidamol is known to possess antithrombotic and additionally antimetastatic properties.

In one aspect, the invention includes, an improved drug-delivery endoprosthetic device for insertion at a vascular site via catheter placement at the site, comprising:
(a) a structural member into the upper and/or lower surface of which one or more micro-deepenings are engraved and/or on which a polymer member is carried, for co-expansion with the polymer member from a contracted state to an expanded state when the device is exposed to said stimulus,
(b) optionally a polymer member capable of expanding from a contracted to a stable, expanded state when the polymer member is exposed to a selected stimulus,
wherein the device can be delivered from a catheter, with the structural and the optional polymer members in their contracted states, and is adapted to be held in a vessel at the vascular target site by radial pressure against the wall of the vessel, with the structural and the optional polymer members in their expanded states; and
wherein the micro-deepenings of said structural member and/or said polymer member comprise a pharmaceutical composition containing one or more active ingredients selected from the group consisting of agents to inhibit or at least reduce excessive proliferation of vessel wall cells, agents to enhance the downstream perfusion of tissue, agents to promote and/or to enhance the neo-formation of capillaries, agents designed to modulate the amount or activity of coagulation factors, agents to reduce the amount of Thrombin- and / or Fibrin-formation, embedded therein for release from the member, with such in its expanded state, the improvement wherein is that said pharmaceutical composition comprises at least one pyrimido-pyrimidine compound selected from dipyridamole, mopidamol and the pharmaceutically acceptable salts thereof, optionally in combination with one or more other antithrombotic agents, agents to enhance lysis of fibrin, agents to locally arrest cell proliferation in a reversible or in an irreversible manner, a gene transfer protein, an inhibitor of metallo-protease, a statin, an antifungal antibiotic such as rapamycin, an ACE inhibitor, an Angiotensin II antagonist, an ADP receptor inhibitor, a Ca-antagonist and/or a lipid-lowering agent.
The device may include a shape-memory polymer member capable of expanding from a contracted state to a stable, radially expanded state when the polymer member is exposed to a selected stimulus.

In one embodiment, the polymer member is composed of a shape-memory polymer responsive to a thermal stimulus at a temperature between about 25°-100° C.

The polymer member is coextensive with the structural member, or, in other embodiments, the polymer member encases the structural member and, in its contracted state, is effective to restrain the structural member in its contracted state.

In one embodiment, the thermally-responsive polymer member is formed of a memory polymer having a thermally-activated polymer-state transition which is a melting point of the polymer; a glass-transition of the polymer; a liquid crystal transition; or a local mode molecular transition. Such a polymer can be an acrylate-containing or a methacrylate-containing polymer.

In another embodiment, the structural member expands in response to a heat stimulus or radial force. Preferably, such a structural member composed of a metal or alloy such as Nitinol, stainless steel, titanium, tantalum, cobalt, platinum, and iridium.

In a preferred embodiment, the structural member is composed of a shape-memory alloy for radial expansion at a critical temperature by activating a heat-recoverable memory diameter and the device is heated to the critical temperature. In another preferred embodiment, the structural member is composed of a heat-activated, shape memory polymer. In another preferred embodiment, the structural member is composed of a metal and designed for self-expansion.

In another preferred embodiment, the active ingredients can be eluted simultaneously or in a specified sequence and with different eluation characteristics.

Preferably dipyridamole or a pharmaceutically acceptable salt thereof can be used alone in a monopreparation or in combination with other antithrombotic agents for the reduction of vascular smooth muscle cell proliferation induced by stents. Most preferred is the utilization of dipyridamole in the presence of a dissolution mediation agent, preferably an organic acid or a derivative thereof, in particular tataric acid or cyclohexanedicarboxylic acid anhydride (CHD). Most preferred is a composition comprising 1 part per weight dipyridamole and 0.1 to 50, preferably 0.5 to 10, in particular 0.8 to 5 part per weight tataric acid or cyclohexanedicarboxylic acid anhydride.

It is of advantage to maintain a tissue level which corresponds to a plasma level of dipyridamole or mopidamol of about 0.2 to 5 µmol/L, preferably of about 0.4 to 5 µmol/L, especially of about 0.5 to 2 µmol/L or particularly of about 0.8 to 1.5 µmol/L. This can be achieved by direct loading of the polymer member of the stent or dipyridamole retard, instant or the parenteral formulations on the market, the retard formulations being preferred, for instance those available under the trademark Persantin^{®}, or, for the combination therapy with low-dose ASA, using those formulations available under the trademark Asasantin^{®} or Aggrenox^{®}. Dipyridamol retard formulations are also disclosed in EP-A-0032562, instant formulations are disclosed in EP-A-0068191 and combinations of ASA with dipyridamole are disclosed in EP-A-0257344. In case of mopidamol also oral retard, instant or a parenteral formulations can be used, e.g. those disclosed in GB 1,051,218 or EP-A-0,108,898, retard formulations being preferred.

In another preferred embodiment the depot of active ingredient(s) of the stent according to the present invention may be reloaded with dipyramidole and/or an additional active ingredient in vivo to maintain bowel tissue level at a constant level with minimal variations. Preferably such stents can be reloaded with active ingredient(s) wherein the polymer member comprises hydrogels.

In addition to the implanted stent dipyridamole or mopidamol may be administered in a daily dosage of 50 to 900 mg, preferably 100 to 480 mg, most preferred 150 to 400 mg. For long-term treatment it is of advantage to administer repeated doses such as a dose of 25 mg dipyridamole retard or any other instant release formulation three or four times a day. For parenteral administration dipyridamole could be given in a dosage of 0.5 to 5 mg/kg body weight, preferably 1 to 3.5 mg/kg body weight, during 24 hours.

Dipyridamole or mopidamol in combination with low-dose ASA may be administered orally in a daily dosage of 10 to 50 mg of ASA together with 100 to 600 mg of dipyridamole or mopidamol, preferably 160 to 480 mg of dipyridamole or mopidamol, for instance in a weight ratio between 1 to 5 and 1 to 12, most pre-ferred a weight ratio of 1 to 8, for instance 50 mg of ASA together with 400 mg of dipyridamole or mopidamol.

Other antithrombotic compounds may be contained in the stent at 0.1 to 100 times, preferably at 0.3 to 30 times, most preferred at 0.3 to 10 times the clinically described dose (e.g. Rote Liste^{®} 1999; fradafiban, lefradafiban: EP-A-0483667), together with a daily dosage of 50 to 900 mg, preferably 100 to 480 mg, most preferred 150 to 400 mg of dipyridamole or mopidamol.

For combination treatment using dipyridamole or mopidamol together with ACE inhibitors any ACE inhibitor known in the art would be suitable, e.g. benazepril, captopril, ceronapril, enalapril, fosinopril, imidapril, lisinopril, moexipril, quinapril, ramipril, trandolapril or perindopril, using dosages corresponding to those known in the art, for instance as described in Rote Liste^{®} 1999, Editio Cantor Verlag Aulendorf.

For combination treatment using dipyridamole or mopidamol together with Angiotensin II receptor antagonists any Angiotensin II receptor antagonist known in the art would be suitable, e.g. the sartans such as candesartan, eprosartan, irbesartan, losartan, telmisartan, valsartan, olmesartan or tasosartan, using dosages corresponding to those known in the art, for instance as described in Rote Liste^{®} 1999, Editio Cantor Verlag Aulendorf.

For combination treatment using dipyridamole or mopidamol together with Ca-antagonists any Ca-antagonist known in the art would be suitable, e.g. nifedipine, nitrendipine, nisoldipine, nilvadipine, isradipine, felodipine or lacidipine, using dosages corresponding to those known in the art, for instance as described in Rote Liste^{®} 1999, Editio Cantor Verlag Aulendorf.

For combination treatment using dipyridamole or mopidamol together with statins any statin known in the art would be suitable, e.g. lovastatin, simvastatin, pravastatin, fluvastatin, atorvastatin or cerivastatin, using dosages corresponding to those known in the art, for instance as described in Rote Liste^{®} 1999, Editio Cantor Verlag Aulendorf.

The additional drug embedded in the polymer member is, for example, an anticoagulant, an antiproliferative agent, a vasodilator, a nitrate, an antioxidant, antisense oligonucleotide, an antiplatlet agent, or a clot dissolving enzyme. In a preferred embodiment, the drug is the anticoagulant heparin.

In one embodiment, the polymer member is carried on the structural member and is secured thereon by an adhesive. The adhesive can be, for example, a biopolymer, such as a protein or a peptide. The adhesive can also be prepared from a synthetic polymer which swells or is soluble in water, and exemplary polymers are given below. In a preferred embodiment, the adhesive is prepared from heparin.

These and other objects and features of the invention will be more fully appreciated when the following detailed description of the invention is read in conjunction with the accompanying drawings.

### BRIEF DESCRIPTION OF THE DRAWINGS

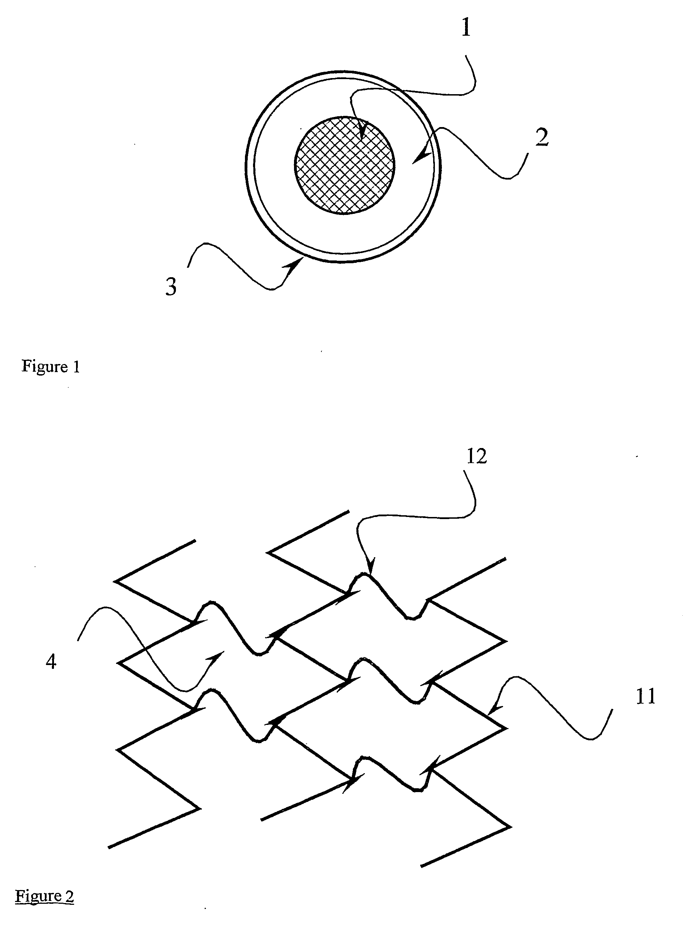

FIG. 1 illustrates cross-section of a strut of an endoprosthetic device in accordance with one embodiment of the invention, where the structural member (1) is encased by the polymer member (2) containing dipyridamole, which may be coated by a second optional polymer member (3) which allows to influence the release properties of the active ingredients.

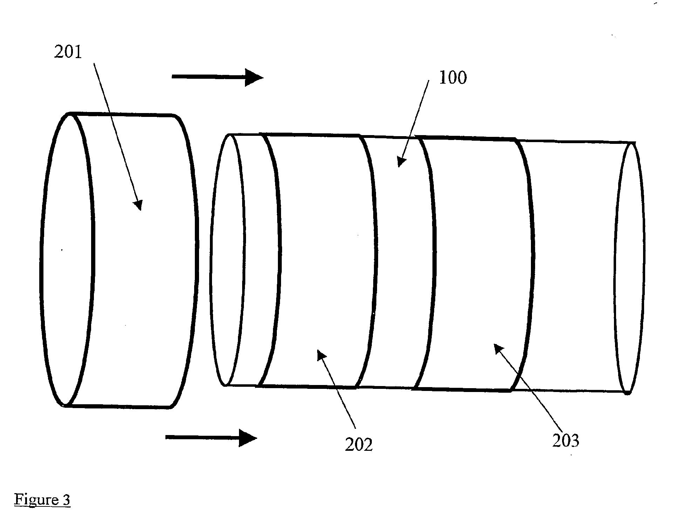

FIG. 2 illustrates an example of a 2-dimensional network of an endoprosthetic device in accordance with one embodiment of the invention, wherein zig-zag shaped struts (11) are cross-linked with additional struts (12). The cell (4) formed from (11) and (12) allows to avoid that side branches of the vessel are closed by the stent.
FIG. 3 illustrates an endoprosthetic device in accordance with one embodiment of the invention, where the 2-dimensional network of struts forms a cylinder shaped stent (100), the surface of which which is partially coated by additional rings (202), (203) comprising additional active ingredients. Another ring (201) may be attached to the tube (100). The rings themselves may consist of mashes allowing several of them to be displayed on top of each other, without blocking side branches or bifurcations of the vessel.

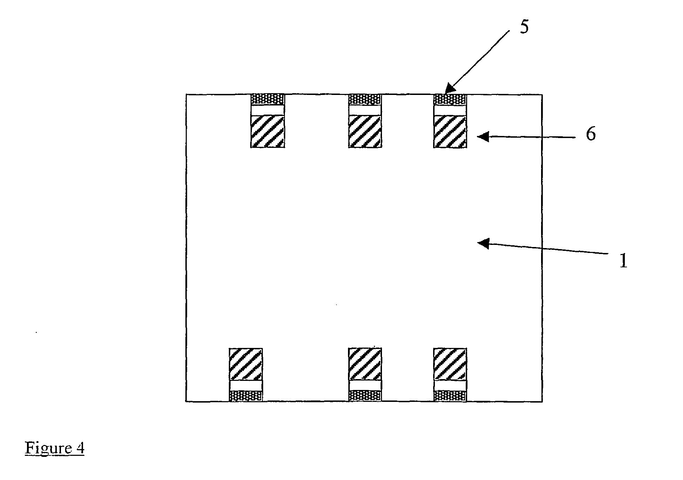

FIG. 4 illustrates a cross-section of a strut of an endoprosthetic device in accordance with one embodiment of the invention, where the structural member (1) is engraved with micro-deepenings or grooves along the strut containing active drug such as dipyridamole or others. Different micro deepenings (pockets) may contain different drugs as well as different coatings to allow release with different pharmacokinetics. (6), which may be coated by an optional polymer layer (5) which allows to influence the release properties of the active ingredients.

### DETAILED DESCRIPTION OF THE INVENTION

The endoprosthetic device of the present invention, also referred to herein as a stent, is designed for insertion at a vessel target site via a catheter. As will be described, the low-profile, self-restraining stent is designed for expansion in response to a stimulus and for administration of a therapeutic compound for release at the target site.

In its most broad aspect, the device is composed of a structural member having engraved micro-deepenings and/or an optional polymer member. The two members are designed for coexpansion, where, in one embodiment, the members are coextensive and, in another embodiment, the polymer member encases the structural member. Each of these embodiments will be described below in detail.
In a first embodiment of the device a structural member is encased by the polymer member. The device is generally tubular or cylindrical in shape. A structural member gives mechanical strength to the device and, importantly, carries on its outer surfaces either and/or a polymer member. In accordance with this first embodiment the polymer member encases and/or surrounds the structural member.

In a particularly preferred embodiment the polymer member comprises two or more layers of different polymers having different elution properties on one structural member.

In a second embodiment of the device a structural member is engraved with micro-deepenings. The device is generally tubular or cylindrical in shape. A structural member gives mechanical strength to the device and, importantly, carries on its outer surfaces said micro-deepenings.

The micro-deepenings are engraved on the structural member for example by laser etching techniques, as will be described below. As will be described below in more detail, the micro-deepenings are filled with a pharmaceutical composition comprising e.g. dipyramidole and are covered with a polymer coating, subsequently, the device is expanded by, for example, exposing the structural member to a heat stimulus to activate a material transition for recovery to a memory state or by a radial force, such as provided by inflation of a balloon on a catheter.

### A. The Structural Member

The structural member of the device is formed preferably of a metal or an alloy, including shape-memory alloys. Exemplary metals include stainless steel, titanium, nickel, tantalum, cobalt, platinum and iridium. Exemplary alloys include alloys of these metals, Cu-Zn-Al, Cu-Al-Ni and shape-memory alloys ofNi-Ti alloys, known under the name Nitinol, Bimetal or Memotal.

Most preferred are biodegradable structural member combined with biodegradable polymer members having different biodegradation profiles.

The structural member of the device may also be formed from a polymer, in particular a shape-memory polymer, and exemplary polymers are given below.

The structural member can take a wide variety of geometries or configurations, such as those described herein, and those known in the art. Commercially available stents suitable for use as the structural member include Johnson & Johnson's Interventional Stent System, a low-profile stent from Arterial Vascular Engineering, the Cook Stent, from Cook Cardiology Co., the BX^{™} stent, from Cordis and the Cypher^{™}, Sirolimus (Sacrolimus) eluting stent from Cordis.

In a particular preferred embodiment micro-deepenings are engraved into the upper and/or lower surface of the structural element. These micro-deepenings contain a pharmaceutical composition, which comprises dipyramidole and / or other active drugs.

The micro-deepenings can take a wide variety of geometries or configurations, such as those described herein, and those known in the art. Most preferred are micro-channels, which extend over the complete surface of the strut or micro-wholes, which are plotted in certain designs on the surface of the strut. These can be engraved into the surface of the structural elements with the aid of laser etching techniques. As a rule the micro-deepenings cover up to 40 %, preferably 5 to 35 %, in particular 10 to 20 % of the upper and/or lower surface of the structural element. Up to 80 %, preferably 30 to 70 %, in particular 40 to 60 % of the height of the perimeter of the structural element can be engraved in order to form micro-deepenings without destabilization of the strut.

### B. The Polymer Member

The polymer member may be of pave extension type or of shape-memory type. Both types are suitable to provide a carrier basis for a variety of organic and inorganic compounds. The carrier may be reloaded or recharged, in the event that the plasma and/or tissue level drops below a certain, desired level.

The polymer member of the device is formed from a shape-memory polymer formulated to have a polymer-state transition that responds to a selected stimulus. Upon exposure to the stimulus, the polymer transition is activated and the polymer member moves from a contracted, small-diameter state to an expanded, larger-diameter state.

Shape-memory polymers suitable for use in the present invention include, for example, those described in U.S. Pat. No. 5,163,952, which is incorporated by reference herein. In particular, the shape-memory polymer is a methacrylate-containing or an acrylate-containing polymer, and exemplary formulations are given below.

As discussed above, the shape-memory polymer member is characterized in that it will attempt to assume a memory condition in response to a stimulus which activates a polymer transition. Such a stimulus can be (i) adsorption of heat by the polymer, (ii) adsorption of liquid by the polymer, (iii) a change in pH in the liquid in contact with the polymer or (iv) absorption of light.

Polymers responsive to heat are those that undergo a thermal transition at a critical temperature. For example, such a thermal transition can be a crystalline melting point of the either the main chain or a side chain of the polymer, preferably between about 25°-100° C.; a glass-transition at a temperature of between 25°-100° C., more preferably between 25°-80° C.; a liquid-crystal phase (mystifies) temperature transition; or a local mode molecular transition.

Polymers responsive to adsorption of a liquid are formulated by incorporating in the polymer a hydrophilic material, such a N-vinyl pyrrolidone. Typically, upon exposure to an aqueous medium the N-vinyl pyrrolidone absorbs water and swells, causing expansion of the polymer.

Polymers responsive to a change in pH are formulated by incorporating pH sensitive materials into the polymer, such as methacrylic acid or acrylic acid. Typically, these polymers swell in response to a change in ionic environment, for movement between a small, contracted state and a larger, expanded state.

In a preferred embodiment of the invention, the polymer member is prepared from a polymer that is sensitive to heat. Typically, these polymers are thermoplastic polymers which soften and take on a new shape by the application of heat and/or pressure. These polymers can be crosslinked to varying degrees so that the polymer will soften with heat but not flow. As discussed above, preferably, the shape-memory polymer for use in forming the structural member of the device is a heat-sensitive, polymer, and in particular a methacrylate-containing or an acrylate-containing polymer.

An exemplary methacrylate-containing memory polymer is prepared by mixing the monomers methyl methacrylate, polyethyleneglycol methacrylate, butylmethacrylate in a 2:1.5:1 ratio. A crosslinker, such as hexanedioldimethacrylate, and a thermal or UV initiator, such as benzoin methyl ether or azobisisobutylnitrile (AIBN). The monomers can be polymerized into a polymer for extrusion in a conventional extruder to provide a length of a tubular structure or a flat sheet, which are cross-linked by exposure to UV light, high energy electrons, gamma radiation or heat. The monomers can also be polymerized in a transparent spinning tube to form a tubular structure.

In experiments performed in support of the present invention, described below, polymer members were formed from the monomers methyl methacrylate, polyethyleneglycol methacrylate, and butylmethacrylate. The monomers were crosslinked using hexanedioldimethacrylate and the polymerization was initiated using Darocur.
Mother exemplary thermoplastic polymer is polyethylene oxide, a heterochain thermoplastic with a crystalline melting point around 65° C. Polyethylene oxide can be crosslinked using a multifunctional acrylate or methacrylate, such as triallylisocyanurate. Thermoplastic blends are also suitable memory polymers, such as blends of polyethylene oxide with methylmethacrylate, polyethylene, polycaprolactone, or trans-polyoctenamer (Vestenamer®). Typically, between 10-90%, preferably 30-70%, of polyethylene oxide is present in the blends. The blends can be crosslinked using conventional multifunctional crosslinkers.
Other preferred polymers are those prepared by condensation polymerization and free radical, or addition, polymerization. Condensation polymers are those in which the molecular formula of the repeat unit of the polymer chain lacks certain atoms present in the monomer from which it was formed, or to which it can be degraded. Exemplary condensation polymers include polyester, polyanhydride, polyamide, polyurethane, cellulose, polysiloxane.
Radical chain, or addition polymers are those in which a loss of a small molecule does not take place, as in condensation polymers. Polymers formed by addition polymerization include polyethylene, polymethyl methacrylate, polyvinyl chloride, and polyacrylonitrile.

### C. Therapeutic Agent

The endoprosthetic device of the invention includes one or more therapeutic agents, at least one of which being dipyridamole or mopidamol, contained in the micro-deepenings and/or embedded in one or more polymer members for release at the target site. The drugs may be filled into the micro-deepenings by immersing the structural member into a composition comprising the drug and optional evaporation of volatile components. Thereupon the micro-deepenings may be covered by immersing the structural member into a composition comprising a polymerizable compound, optional evaporation of volatile components and heating or irradiation.

Alternatively, the drugs are incorporated into the polymer member by passive diffusion after fabrication of the member, or more preferably, by addition of the drug to the polymer prior to extrusion of the polymer member or prior to polymerization of the member.

Exemplary additional drugs include heparin to prevent thrombus formation; an antiproliferative agent, such as methotrexate; a vasodilator, such as a calcium channel blocker; a nitrate; antiplatlet agents, such as ticlopidine, abciximab (ReoPro.TM.), Integrelin.TM.; clot dissolving enzymes, such as tissue plasminogen activator; antisense oligonucleotides; pro-urokinase; urokinase; streptokinase; antioxidants, such as vitamin E and glutathione; finasteride (Proscar®) for treatment of benign prostatic hyperplasia; metalloproteinase, statine, cyclosporine, second and third generation of immuno-suppressants, FK 540, estrogen-mediated inhibitors of neointima formation; nitric oxide releasing compounds, such as n'-dimethylhexane diamine and 1-arginines; virus-mediated gene transfer agents; antimitogenic factors and antiendothelin agents.

### D. Device Configurations

The structural and polymer members of the device can take any number of geometric configurations.

Preferably the structural member in the device is a self-expanding stent, where the structural member in its contracted state is under tension and in the absence of a restraining member, will expand to its larger diameter state. The optional polymer member acts as a restraining member for the structural member. The polymer member, formed of a shape-memory polymer, is self-restraining, e.g., it maintains its small-diameter condition until the polymer transition is activated. This feature of the device is beneficial in maintaining a low device profile.
Expansion of the device may be achieved by exposing the polymer member to a stimulus, such as heat, to activate the polymer transition. As the polymer member expands, the structural member is no longer restrained and coexpands with the polymer member.

It will be appreciated that the device can be formed of a variety of materials and geometries. For example, the structural member can be either a polymer or a metal and the flat sheet configuration may be provided with slots, openings or gaps. It will further be appreciated that the structural member and the polymer members can have different geometries.

### E. Device Preparation

In preparing the endoprosthetic device of the present invention, the optional polymer member and the structural members are each prepared and then brought together to form the device. The selection of material for each of the device members depends in part of the configuration of each member and on whether the polymer member encases the structural member or is coextensive with the structural member.

Preferably the polymer member is prepared from a monomer mixture that is polymerized by exposure to UV light. The resulting polymer film or tube has a thermal transition between about 35° - 50° C. and a polymer member is cut, using a precision blade or a laser, to the desired geometry. The polymer member is placed in its small-diameter, contracted state by heating the member above its thermal transition and wrapping the member around an appropriate sized tube or rod. The member is cooled to set the shape and removed from the tube. The member is then slipped over a structural member, typically a metal or metal alloy stent purchased from commercially available sources or prepared according to known methods. For example, the Johnson & Johnson Interventional System Stent, having a slotted tube design, can be used, as can a Cook Stent, from Cook Cardiology. It will be appreciated that the polymer member can be wrapped directly around the structural member rather than around a rod or tube.

Alternatively, the polymer member is prepared from a polymer mixture which is heated and blended in a conventional extruder for extrusion to coat the struts of the structural member or to form the desired geometry, either a cylindrical tube or a rectangular strip. In the Example, the polymer member is prepared from polyoctenylene and polyethylene glycol and crosslinked with triallyl isocyanurate. Other polymers, such as polyethylene, are also suitable.

After extrusion of a separate polymer member, the polymer member is cut the appropriate length, converted into a mesh and/or slipped over the structural member or cowound with the structural member, depending on the geometry of each member. For example, a structural member having a flat rectangular shape can be prepared from Nitinol, available from Shape Memory Applications (Santa Clara, Calif.).

The structural member and the polymer member, having the same geometry are heated to above their respective transition temperatures and cowound around a stainless steel rod, having a diameter selected according to the desired final stent size. The members are cooled while being restrained in the contracted shape around the rod, to form the device.
In the case where the structural member and the polymer member of the device are both formed of a polymer, the device may also include a radio-opaque material, such as gold, stainless steel, platinum, tantalum, bismuth, metal salts, such as barium sulfate, or iodine containing agents, such as OmniPaque® (Sanofi Winthrop Pharmaceuticals). The radio-opaque material may be incorporated into the polymer prior to extrusion of device members, or a radio-opaque coating may be applied to one or both of the members. The radio-opaque material provides a means for identifying the location of the stent by x-rays or other imaging techniques during or after stent placement. Preparation of a polymer member having regions of radio-opacity, provided by gold particles dispersed in the polymer member, is described in

### F. Method of Use

As discussed above, the endoprosthetic device of the present invention is placed at a target vascular site by a transluminal angioplasty catheter. The catheter is introduced over a conventional guidewire and the stent is positioned within the target site using, for example, fluoroscopic imaging.

Once the stent is properly positioned, a balloon is filled with a liquid to stimulate the polymer-state transition of the polymer member. As discussed above, the polymer transition may be thermally induced, may be activated by a change in pH or adsorption of a liquid or may be light induced by fiber optic. Upon exposure to the stimulus, the stent expands from its small-diameter state toward its memory condition. For example, a stent having a thermally-activated polymer transition is stimulated to expand by filling the catheter balloon with a heated liquid, such as a contrast agent heated to between about 40°-100° C. Heat from the liquid is adsorbed by the polymer member. The catheter itself may be specifically designed for injection of a heated liquid and for better heat transfer. For example, the catheter may have a double lumen for recirculation of the heated liquid in the balloon region of the catheter.

The stimulus may also be a pH stimulus or a liquid stimulus, where a buffer solution of a selected pH is introduced into the balloon. Small openings in the balloon, introduced prior to placement of the stent around the balloon, would allow the liquid to contact the stent.
In a preferred embodiment, the stimulus is a thermal stimulus, and a heated liquid is introduced into the balloon. Heat from the liquid is conducted convectively to the polymer stent, raising the temperature of the stent to its thermal transition, such as a glass transition temperature of between about 25°-100° C., more preferably between 25°-80° C., and most preferably between 35°-70° C. The polymer member responds to the stimulus by moving toward its memory condition. The structural member coexpands with the polymer member, either in response to the thermal stimulus, the radial force of the inflated balloon or by the self-expanding design of the structural member. Expansion of the device continues until the members are constrained by the vessel walls. Once the stent is fully deployed with the segments in their expanded condition, the catheter may be withdrawn over the guidewire, and the guidewire removed.

### EXAMPLES

The following examples detail preparation of endoprosthetic devices in accordance with the invention and are intended to be exemplary and in no way to limit the scope of the invention.

### Example 1

### Test model

Incorporation of BrdU instead of thymidine into the DNA, measurement with anti-BrdU - POD antibody (Cell proliferation ELISA, BrdU; obtained from Roche Diagnostics, Mannheim, Germany)
The following results have been obtained using this test with the stents loaded with dipyramidol according to the present invention:
IC50: 0.1-0.3 µM / L dipyridamol; muscle cells stimulated with PDGF-BB
IC50: 4-10 µM / L dipyridamol; with freshly prepared medium
IC50: 1-3 µM / L dipyridamol; muscle cells without stimulation

### Example 2

A stent has been prepared according to the method disclosed in US Patent US 5,674,242. The polymer member thereof has been made from a mixture of 1 to 10 g dipyramidole, 1 to 50 g cyclohexanedicarboxylic anhydride and 50 to 200 g of different methacrylate monomers.

The stent shows the following properties upon 16 hours of incubation:
100 % inhibition of DNA synthesis; strong release of dipyridamol.

### Example 3

A stent has been prepared according to the method disclosed in US Patent US 5,674,242. The polymer member thereof has been made from a mixture of 1 to 10 g dipyramidole, 2 to 10 g tartaric acid and 50 to 200 g of different methacrylate monomers.

The stent shows the following properties upon 16 hours of incubation:
96 % inhibition of DNA synthesis; strong release of dipyridamol.

### Example 4

A stent has been prepared according to the method disclosed in US Patent US 5,674,242. The polymer member thereof has been made from a mixture of 1 to 10 g dipyramidole and 50 to 200 g of different methacrylate monomers.

The stent shows the following properties upon 16 hours of incubation:
74 % inhibition of DNA synthesis; weak release of dipyridamol.

## Claims

1. An improved drug-delivery endoprosthetic device for insertion at a vascular site via catheter placement at the site, comprising:
(a) a structural member, into the upper and/or lower surface of which one or more micro-deepening's are engraved and/or on which a polymer member is carried, for co-expansion with the polymer member from a contracted state to an expanded state when the device is exposed to said stimulus,
(b) optionally a polymer member capable of expanding from a contracted to a stable, expanded state when the polymer member is exposed to a selected stimulus,
wherein the device can be delivered from a catheter, with the structural and the optional polymer members in their contracted states, and is adapted to be held in a vessel at the vascular target site by radial pressure against the wall of the vessel, with the structural and the optional polymer members in their expanded states; and
wherein the micro-deepening's of said structural member and/or said polymer member comprise a pharmaceutical composition containing one or more active ingredients selected from the group consisting of agents to inhibit or at least reduce excessive proliferation of vessel wall cells, agents to enhance the downstream perfusion of tissue, agents to promote and/or to enhance the neo-formation of capillaries, agents designed to modulate the amount or activity of coagulation factors, agents to reduce the amount of Thrombin- and / or Fibrin-formation, embedded therein for release from the member, with such in its expanded state, the improvement wherein is that said pharmaceutical composition comprises at least one pyrimido-pyrimidine compound selected from dipyridamole, mopidamol and the pharmaceutically acceptable salts thereof in the presence of a dissolution mediation agent, optionally in combination with one or more other antithrombotic agents, agents to enhance lysis of fibrin, agents to locally arrest cell proliferation in a reversible or in an irreversible manner, a gene transfer protein, an inhibitor of metallo-protease, a statin, an antifungal antibiotic such as rapamycin, an ACE inhibitor, an Angiotensin II antagonist, an ADP receptor inhibitor, a Ca-antagonist and/or a lipid-lowering agent.

2. The device according to claim 1, wherein the dissolution mediation agent agent is an organic acid or a derivative thereof.

3. The device according to claim 1 or 2, wherein the dissolution mediation agent is tartaric acid or cyclohexanedicarboxylic acid anhydride.

4. The device according to claims 1-3 wherein the different active ingredients can be eluted simultaneously.

5. The device according to any of the preceding claims, wherein the different active ingredients can be eluted in a specified sequence and with different eluation characteristics.

6. The device according to any of the preceding claims, wherein the pyrimido-pyrimidine is dipyridamole.

7. The device according to any of the preceding claims, comprising the pyrimido-pyrimidine in an amount so that a plasma level of about 0.2 to 5 µmol/L thereof is maintained.

8. The device according to any of the preceding claims, **characterized in that** the pyrimido-pyrimidine is administered in a dosage of 0.5 to 5 mg/kg body weight during 24 hours.

9. The device of according to any of the preceding claims, wherein said polymer member is composed of a shape-memory polymer responsive to a thermal stimulus at a temperature from about 25° to 100° C.

10. The device according to any of the preceding claims, wherein said polymer member is coextensive with said structural member.

11. The device according to any of the preceding claims, wherein said polymer member encases said structural member and, in its contracted state, is effective to restrain said structural member in its contracted state.

12. The device according to any of the preceding claims, wherein said thermally-responsive polymer member is formed of a memory polymer having a thermally-activated polymer-state transition selected from the group consisting of:
(a) a melting point of the polymer;
(b) a glass-transition of the polymer;
(c) a liquid crystal transition; and
(d) a local mode molecular transition.

13. The device of claim 12, wherein said polymer member is an acrylate-containing or a methacrylate-containing polymer.

14. The device according to any of the preceding claims, wherein said structural member is responsive to a stimulus selected from the group consisting of heat and radial force.

15. The device according to any of the preceding claims, wherein said structural member is a metal or alloy selected from the group consisting of Nitinol, stainless steel, titanium, tantalum, cobalt, platinum, and iridium.

16. The device according to any of the preceding claims, wherein said structural member is composed of a shape-memory alloy for radial expansion at a critical temperature by activating a heat-recoverable memory diameter and said device is heated to said critical temperature.

17. The device according to claims 1 to 14, wherein said structural member is composed of a heat-activated, shape memory polymer.

18. The device according to claims 1 to 16, wherein said structural member is composed of a metal and designed for self-expansion.

19. The device of according to any of the preceding claims, wherein said pharmaceutical composition comprises dipyridamole or a pharmaceutically acceptable salt thereof, in combination with heparin and/or Clopidogrel.

20. The device according to any of the preceding claims, wherein said polymer member is carried on said structural member by attaching said polymer member to said structural member by an adhesive.

21. The device of claim 20, wherein said adhesive is a biopolymer selected from the group consisting of proteins and peptides.

22. The device of claim 20 or 21, wherein said adhesive is prepared from a synthetic polymer which swells or dissolves in water.

23. The device according to any of the preceding claims, wherein the micro-deepening's cover up to 40 % of the upper and/or lower surface and are engraved for up to 80 % of the height of the perimeter of the structural element.

## Patentansprüche

1. Verbesserte endoprothetische Vorrichtung zur Arzneimittelzuführung zum Einsetzen an einer vaskulären Stelle mittels Katheterpositionierung an dieser Stelle, umfassend:
(a) ein strukturelles Bauteil, in dessen obere und/oder untere Oberfläche ein oder mehrere Mikrovertiefungen eingraviert bzw. eingeprägt sind und/oder auf denen ein Polymerbauteil getragen wird, zur Co-Expansion mit dem Polymerbauteil aus einem kontrahierten Zustand in einen expandierten Zustand, wenn die Vorrichtung dem Stimulus ausgesetzt wird,
(b) gegebenenfalls ein Polymerbauteil, das in der Lage ist, sich aus einem kontrahierten Zustand in einen stabilen expandierten Zustand auszudehnen, wenn das Polymer einem ausgewählten Stimulus ausgesetzt wird,
wobei die Vorrichtung mit den strukturellen und den optionalen Polymerbauteilen in ihren kontrahierten Zuständen durch einen Katheter ausgesetzt werden kann, und angepasst ist, um in einem Gefäß an der vaskulären Zielstelle durch radialen Druck gegen die Gefäßwand gehalten zu werden, mit den strukturellen und optionalen Polymerbauteilen in ihren expandierten Zuständen; und
worin die Mikrovertiefungen des strukturellen Bauteils und/oder des Polymerbauteils eine pharmazeutische Zusammensetzung umfassen, die ein oder mehrere Wirkstoffe enthält, die ausgewählt sind aus der Gruppe, bestehend aus Mitteln, um die exzessive Proliferation von Gefäßwandzellen zu inhibieren oder wenigstens zu reduzieren, Mitteln zur Verstärkung der stromabwärtigen Perfusion von Gewebe, Mitteln zur Förderung und/oder Verbesserung der Neubildung von Kapillaren, Mitteln, entworfen zur Modulation der Menge oder Aktivität von Koagulationsfaktoren, Mitteln zum Reduzieren der Menge der Thrombin- und/oder Fibrin-Bildung, darin eingebettet zur Freigabe aus dem Bauteil, wenn dieses sich in seinem expandierten Zustand befindet, wobei die Verbesserung darin liegt, dass die pharmazeutische Zusammensetzung mindestens eine Pyrimido-Pyrimidin-Verbindung umfasst, ausgewählt aus Dipyridamol, Mopidamol und den pharmazeutisch akzeptablen Salzen davon, in Gegenwart eines lösungsvermittelnden Mittels, gegebenenfalls in Kombination mit ein oder mehreren anderen antithrombotischen Mitteln, Mitteln zur Verbesserung der Fibrinolyse, Mitteln, um in einer reversiblen oder irreversiblen Art und Weise lokal die Zellproliferation zu hemmen, einem Gentransferprotein, einem Metalloprotease-Inhibitor, einem Statin, einem antifungalen Antibiotikum, wie Rapamycin, einem ACE-Inhibitor, einem Angiotensin-II-Antagonist, einem ADP-Rezeptorinhibitor, einem Ca-Antagonist und/oder einem Fettsenkungsmittel.

2. Vorrichtung nach Anspruch 1, worin das lösungsvermittelnde Mittel eine organische Säure oder ein Derivat hiervon darstellt.

3. Vorrichtung nach Anspruch 1 oder 2, worin das lösungsvermittelnde Mittel Weinsäure oder Cyclohexandicarbonsäureanhydrid darstellt.

4. Vorrichtung nach den Ansprüchen 1 bis 3, worin verschiedene Wirkstoffe gleichzeitig eluiert werden können.

5. Vorrichtung nach irgendeinem der vorhergehenden Ansprüche, worin die verschiedenen Wirkstoffen in einer speziellen Reihenfolge und mit verschiedenen Elutionscharakteristika eluiert werden können.

6. Vorrichtung nach irgendeinem der vorhergehenden Ansprüche, worin das Pyrimido-Pyrimidin Dipyridamol darstellt.

7. Vorrichtung nach irgendeinem der vorhergehenden Ansprüche, umfassend Pyrimido-Pyrimidin in einer Menge, so dass ein Plasmaspiegel von etwa 0,2 bis 0,5 µMol/l hiervon aufrechterhalten wird.

8. Vorrichtung nach irgendeinem der vorhergehenden Ansprüche, **dadurch gekennzeichnet, dass** das Pyrimido-Pyrimidin in einer Dosierung von 0,5 bis 5 mg/kg Körpergewicht während 24 Stunden verabreicht wird.

9. Vorrichtung nach irgendeinem der vorhergehenden Ansprüche, worin das Polymerbauteil aus einem Formerinnerungs-Polymer aufgebaut ist, das auf einen thermischen Stimulus bei einer Temperatur von etwa 25°C bis 100°C reagiert.

10. Vorrichtung nach irgendeinem der vorhergehenden Ansprüche, worin das Polymerbauteil koextensiv mit dem strukturellen Bauteil ist.

11. Vorrichtung nach irgendeinem der vorhergehenden Ansprüche, worin das Polymerbauteil das strukturelle Bauteil umhüllt, und in seinem kontrahierten Zustand wirksam ist, um das strukturelle Bauteil in seinem kontrahierten Zustand zu halten.

12. Vorrichtung nach irgendeinem der vorhergehenden Ansprüche, worin das thermischreaktive Polymerbauteil aus einem Memory-Polymer gebildet wird, das einen thermisch-aktivierten Polymerzustandsübergang aufweist, der ausgewählt ist aus der Gruppe, bestehend aus:
(a) einem Schmelzpunkt des Polymers;
(b) einem Glasübergang des Polymers;
(c) einem Flüssigkristallübergang; und
(d) einem lokalen molekularen Übergang.

13. Vorrichtung nach Anspruch 12, worin das Polymerbauteil ein Acrylat-enthaltendes oder Methacrylat-enthaltendes Polymer darstellt.

14. Vorrichtung nach irgendeinem der vorhergehenden Ansprüche, worin das strukturelle Bauteil auf einen Stimulus reagiert, der ausgewählt ist aus der Gruppe, bestehend aus Wärme und radialer Kraft.

15. Vorrichtung nach irgendeinem der vorhergehenden Ansprüche, worin das strukturelle Bauteil ein Metall oder eine Legierung darstellt, ausgewählt aus der Gruppe, bestehend aus Nitinol, rostfreiem Stahl, Titan, Tantal, Kobalt, Platin und Iridium.

16. Vorrichtung nach irgendeinem der vorhergehenden Ansprüche, worin das strukturelle Bauteil aus einer Formerinnerungs-Legierung zur radialen Expansion bei einer kritischen Temperatur durch Aktivierung eines wärme-rückgewinnbaren Erinnerungsdurchmessers aufgebaut ist und die Vorrichtung auf die kritische Temperatur erhitzt wird.

17. Vorrichtung nach den Ansprüchen 1 bis 14, worin das strukturelle Bauteil aus einem wärmeaktivierten Formerinnerungs-Polymer aufgebaut ist.

18. Vorrichtung nach den Ansprüchen 1 bis 16, worin das strukturelle Bauteil aus einem Metall aufgebaut ist und zur Selbstexpansion entworfen wurde.

19. Vorrichtung nach irgendeinem der vorhergehenden Ansprüche, worin die pharmazeutische Zusammensetzung Dipyridamol oder ein pharmazeutisch akzeptables Salz davon in Kombination mit Heparin und/oder Clopidogrel umfasst.

20. Vorrichtung nach irgendeinem der vorhergehenden Ansprüche, worin das Polymerbauteil vom strukturellen Bauteil durch Anbringen des Polymerbauteils am strukturellen Bauteil durch einen Klebstoff getragen wird.

21. Vorrichtung nach Anspruch 20, worin der Klebstoff ein Biopolymer darstellt, ausgewählt aus der Gruppe, bestehend aus Proteinen und Peptiden.

22. Vorrichtung nach Anspruch 20 oder 21, worin der Klebstoff hergestellt wird aus einem synthetischen Polymer, das in Wasser anquillt oder sich auflöst.

23. Vorrichtung nach irgendeinem der vorhergehenden Ansprüche, worin die Mikrovertiefungen bis zu 40% der oberen und/oder unteren Oberfläche bedecken, und bis zu 80% der Höhe des Umfangs des strukturellen Bauteils eingraviert bzw. eingeprägt sind.

## Revendications

1. Dispositif endoprothétique de délivrance de médicament amélioré destiné à être inséré à un site vasculaire par le biais de la mise en place d'un cathéter au site, comprenant :
(a) un membre structural, dans la surface supérieure et/ou inférieure duquel sont gravées une ou plusieurs micro-dépressions et/ou sur lequel un membre polymère est porté, pour la co-expansion avec le membre polymère d'un état contracté à un état expansé quand le dispositif est exposé audit stimulus,
(b) éventuellement un membre polymère capable de s'expanser d'un état contracté à un état expansé stable quand le membre polymère est exposé à un stimulus choisi,
où le dispositif peut être délivré depuis un cathéter, avec le membre structural et le membre polymère éventuel dans leurs états contractés, et est adapté pour être maintenu dans un vaisseau au site cible vasculaire par une pression radiale contre la paroi du vaisseau, avec le membre structural et le membre polymère éventuel dans leurs états expansés ; et
où les micro-dépressions dudit membre structural et/ou ledit membre polymère comprennent une composition pharmaceutique contenant un ou plusieurs ingrédients actifs choisis dans le groupe consistant en des agents pour inhiber ou au moins réduire la prolifération excessive des cellules de la paroi vasculaire, des agents pour augmenter la perfusion aval de tissu, des agents pour favoriser et/ou augmenter la néo-formation de capillaires, des agents conçus pour moduler la quantité ou l'activité de facteurs de coagulation, des agents pour réduire la quantité de formation de thrombine et/ou de fibrine, inclus à l'intérieur pour la libération depuis le membre, avec celui-ci dans son état expansé, l'amélioration dans laquelle ladite composition pharmaceutique comprend au moins un composé pyrimido-pyrimidine choisi parmi le dipyridamole, le mopidamol et leur sels pharmaceutiquement acceptables en présence d'un agent assurant la médiation de la dissolution, éventuellement en combinaison avec un ou plusieurs autres agents antithrombotiques, agents pour augmenter la lyse de la fibrine, agents pour arrêter localement la prolifération cellulaire d'une manière réversible ou d'une manière irréversible, une protéine de transfert de gène, un inhibiteur de métallo-protéase, une statine, un antibiotique antifongique comme la rapamycine, un inhibiteur d'ACE, un antagoniste d'angiotensine II, un inhibiteur de récepteur d'ADP, un antagoniste de Ca et/ou un agent abaissant les lipides.

2. Dispositif selon la revendication 1 où l'agent assurant la médiation de la dissolution est un acide organique ou un dérivé de celui-ci.

3. Dispositif selon la revendication 1 ou 2 où l'agent assurant la médiation de la dissolution est l'acide tartrique ou l'anhydride d'acide cyclohexanedicarboxylique.

4. Dispositif selon les revendications 1-3 où les différents ingrédients actifs peuvent être élués simultanément.

5. Dispositif selon l'une quelconque des revendications précédentes où les différents ingrédients actifs peuvent être élués dans une séquence spécifiée et avec des caractéristiques d'élution différentes.

6. Dispositif selon l'une quelconque des revendications précédentes où la pyrimido-pyrimidine est le dipyridamole.

7. Dispositif selon l'une quelconque des revendications précédentes comprenant la pyrimido-pyrimidine en une quantité telle qu'un niveau plasmatique d'environ 0,2 à 5 µmol/L de celle-ci est maintenu.

8. Dispositif selon l'une quelconque des revendications précédentes **caractérisé en ce que** la pyrimido-pyrimidine est administrée en une posologie de 0,5 à 5 mg/kg de poids corporel pendant 24 heures.

9. Dispositif selon l'une quelconque des revendications précédentes où ledit membre polymère est composé d'un polymère à mémoire de forme sensible à un stimulus thermique à une température d'environ 25° à 100°C.

10. Dispositif selon l'une quelconque des revendications précédentes où ledit membre polymère est co-extensif avec ledit membre structural.

11. Dispositif selon l'une quelconque des revendications précédentes où ledit membre polymère entoure ledit membre structural et, dans son état contracté, est efficace pour retenir ledit membre structural dans son état contracté.

12. Dispositif selon l'une quelconque des revendications précédentes où ledit membre polymère sensible thermiquement est formé d'un polymère à mémoire ayant une transition d'état de polymère thermiquement activée choisie dans le groupe consistant en :
(a) un point de fusion du polymère ;
(b) une transition vitreuse du polymère ;
(c) une transition cristalline liquide ; et
(d) une transition moléculaire en mode local.

13. Dispositif selon la revendication 12 où ledit membre polymère est un polymère contenant un acrylate ou un polymère contenant un méthacrylate.

14. Dispositif selon l'une quelconque des revendications précédentes où ledit membre structural est sensible à un stimulus choisi dans le groupe consistant en la chaleur et une force radiale.

15. Dispositif selon l'une quelconque des revendications précédentes où ledit membre structural est un métal ou alliage choisi dans le groupe consistant en le Nitinol, l'acier inoxydable, le titane, le tantale, le cobalt, le platine et l'iridium.

16. Dispositif selon l'une quelconque des revendications précédentes où ledit membre structural est composé d'un alliage à mémoire de forme pour l'expansion radiale à une température critique par activation d'un diamètre à mémoire susceptible de reprise thermique et ledit dispositif est chauffé à ladite température critique.

17. Dispositif selon les revendications 1 à 14 où ledit membre structural est composé d'un polymère à mémoire de forme activé par la chaleur.

18. Dispositif selon les revendications 1 à 16 où ledit membre structural est composé d'un métal et conçu pour l'auto-expansion.

19. Dispositif selon l'une quelconque des revendications précédentes où ladite composition pharmaceutique comprend du dipyridamole ou un sel pharmaceutiquement acceptable de celui-ci, en combinaison avec l'héparine et/ou le Clopidogrel.

20. Dispositif selon l'une quelconque des revendications précédentes où ledit membre polymère est porté sur ledit membre structural par fixation dudit membre polymère audit membre structural par un adhésif.

21. Dispositif selon la revendication 20 où ledit adhésif est un biopolymère choisi dans le groupe consistant en les protéines et les peptides.

22. Dispositif selon la revendication 20 ou 21 où ledit adhésif est préparé à partir d'un polymère synthétique qui gonfle ou se dissout dans l'eau.

23. Dispositif selon l'une quelconque des revendications précédentes où les micro-dépressions couvrent jusqu'à 40 % de la surface supérieure et/ou inférieure et sont gravées sur jusqu'à 80 % de la hauteur du périmètre de l'élément structural.
